# EUROPÄISCHE PATENTANMELDUNG

(11) **EP 1 772 731 A2**
(43) Veröffentlichungstag der Anmeldung: **11.04.2007**
(21) Anmeldenummer: 06016977.8
(22) Anmeldetag: 15.08.2006
(51) Int. Cl.: G01N 33/50

(54) **Integrierte Sensoranordnung**

(30) Priorität: 07.10.2005 DE 102005048473
(71) Anmelder: Micronas GmbH, 79108 Freiburg i. Br. (DE); Micronas Holding GmbH, 79108 Freiburg (DE)
(72) Erfinder: Lehman, Mirko Dr. rer. nat., 79117 Freiburg (DE); Sieben, Ulrich, Dr. rer. nat., 79279 Vörstetten (DE); Klapproth, Holger, Dr., 79108 Freiburg (DE)
(74) Vertreter: Huwer, Andreas

(57) **Zusammenfassung**

Eine integrierte Sensoranordnung (1) hat ein Substrat (2), mindestens ein Sensorelement (3) und eine in das Substrat (2) integrierte, mit dem Sensorelement (3) verbundene oder verbindbare Signalverarbeitungseinrichtung (4). Eine Schicht, die eine Messoberfläche aufweist, ist derart lösbar mit dem Substrat (2) verbindbar, dass, wenn die Schicht mit dem Substrat (2) verbunden ist, ein von der Messoberfläche ausgehendes Signal mit der Signalverarbeitungseinrichtung (4) verarbeitbar ist. Das Substrat (2) hat mindestens ein an die Schicht angrenzendes Durchgangsloch (7), das zum Ansaugen der Schicht an das Substrat (2) mit einer Einrichtung zur Erzeugung eines Unterdrucks verbunden oder verbindbar ist.

## Beschreibung

Die Erfindung betrifft eine integrierte Sensoranordnung mit einem Substrat, mindestens einem Sensorelement und einer in das Substrat integrierten, mit dem Sensorelement verbundenen oder verbindbaren Signalverarbeitungseinrichtung, wobei eine eine Messoberfläche aufweisende Schicht derart lösbar mit dem Substrat verbindbar ist, dass, wenn die Schicht mit dem Substrat verbunden ist, ein von der Messoberfläche ausgehendes Signal mit der Signalverarbeitungseinrichtung verarbeitbar ist.

Eine derartige Sensoranordnung ist aus DE 102 50 495 A1 bekannt. Sie weist als Substrat einen Halbleiterchip auf, in den mehrere optische Sensorelemente und eine Signalverarbeitungseinrichtung integriert sind. Ein Oberflächenbereich des Halbleiterchips ist mit einem optisch transparenten Polymerband abgedeckt, auf dem Rezeptoren angeordnet sind, die für einen zu detektierenden Liganden bindungsspezifisch sind. Zum Nachweis eines an einen Rezeptor gebundenen Liganden wird in Abhängigkeit von dem Bindungsereignis eine optische Strahlung erzeugt, die mit den optischen Sensorelementen detektiert wird. Das Polymerband ist in einer lösbar mit dem Halbleiterchip verbindbaren, auswechselbaren Kassette angeordnet, in der Rollen zum Ab- bzw. Aufwickeln des Polymerbands vorgesehen sind. Das Polymerband verläuft von einer ersten Rolle zu dem Halbleiterchip und von dort weiter zu einer zweiten Rolle. Mittels einer Transporteinrichtung ist das Polymerband in Längsrichtung relativ zu dem Halbleiterchip verschiebbar. Dabei wird es von der ersten Rolle ab- und auf die zweite Rolle aufgewickelt. Das Polymerband mit dem dahinter befindlichen Halbleiterchip grenzt an eine Innenhöhlung einer Messkammer an, die eine Einlass- und eine Auslassöffnung für einen zu untersuchenden, den Liganden enthaltenden Analyten aufweist. Zwischen dem Polymerband und dem Halbleiterchip ist eine Dichtung angeordnet, an die das Polymerband durch einen in der Innenhöhlung herrschenden Überdruck angedrückt wird. Die vorbekannte Sensoranordnung hat sich in der Praxis vor allem deshalb bewährt, weil der Halbleiterchip nach Durchführung der Messung für weitere Messungen wieder verwendet werden kann. Ein Nachteil der Sensoranordnung besteht jedoch noch darin, dass das Erzeugen des Überdrucks mit einem gewissen Aufwand verbunden ist, insbesondere wenn die Messkammer als offener Trog ausgebildet ist oder die Auslassöffnung mit einem offenen Behälter zur Aufnahme des untersuchten Analyts und der für die Erzeugung der optischen Strahlung benötigten Chemikalien verbunden ist und die Förderung des Analyten und/oder der Chemikalien diskontinuierlich erfolgt.

Es besteht deshalb die Aufgabe, eine Sensoranordnung der eingangs genannten Art zu schaffen, die eine einfache Handhabung ermöglicht.

Diese Aufgabe wird dadurch gelöst, dass das Substrat mindestens ein an die Schicht angrenzendes Durchgangsloch aufweist, das zum Ansaugen der Schicht an das Substrat mit einer Einrichtung zur Erzeugung eines Unterdrucks verbunden oder verbindbar ist.

In vorteilhafter Weise kann dadurch das Erzeugen eines Überdrucks an der dem Substrat abgewandten Seite der die Messoberfläche aufweisenden Schicht entfallen, so dass die Vorrichtung gut handhabbar ist. Dennoch wird die Schicht flächig an das Substrat angedrückt und auf diesem fixiert. Dabei kann die Schicht ggf als Dichtung dienen, welche einen zu untersuchenden, an der dem Substrat abgewandten Seite der Schicht befindlichen Anlayt gegen das Substrat abschottet. Da die Schicht mit der Messoberfläche lösbar mit dem Substrat verbunden ist, kann sie auf einfache Weise ausgewechselt werden, um das Substrat nach Durchführung einer Messung für eine weitere Messung weiter zu verwenden. Das Substrat ist bevorzugst ein Halbleiter-Chip, insbesondere ein Silizium-Chip. Das mindestens eine Durchgangsloch kann beispielsweise durch Bohren oder Ätzen in das Substrat eingebracht werden.

Bei einer zweckmäßigen Ausgestaltung der Erfindung erstreckt sich das mindestens eine Durchgangsloch orthogonal zur Oberfläche des Substrates. Das Substrat lässt sich dann kostengünstig mit an sich bekannten Methoden der Halbleiterfertigung herstellen.

Die vorstehend genannte Aufgabe kann auch dadurch gelöst werden, dass die Vorrichtung Mittel zum Erzeugen von elektrostatischen und/oder magnetischen Kräften zum Fixieren der Schicht auf dem Substrat aufweist. Dabei können diese Mittel insbesondere Permanentmagnete aufweisen, die auf und/oder neben dem Substrat und/oder an der Rückseite des Substrats angeordnet sind und mit einem ferromagnetisch leitenden Bereich der die Messoberfläche aufweisenden Schicht in einem Magnetkreis angeordnet sind. Die magnetischen Kräfte können aber auch durch eine von einem elektrischen Strom durchflossene Induktivität erzeugt werden, die auf, in, unter oder neben dem Substrat angeordnet sein kann.

Die Vorrichtung weist als Sensorelement bevorzugt mindestens einen für eine chemische und/oder physikalische Größe empfindlichen Sensor, insbesondere einen amperometrischen, potentiometrischen, magnetischen, optischen , Druck- und/oder Beschleunigungssensor auf Insbesondere kann das mindestens eine Sensorelement ein IDES, ISFET und/oder eine Clark-Elektrode sein. Mittels des elektrischen Kontaktelements können elektrische Signale, die z.B. von lebenden biologischen Zellen oder Biomolekülen ausgesendet werden, die an der Messoberfläche angewachsen sind, detektiert und über elektrische Leitungen zu der Signalverarbeitungseinrichtung weitergeleitet werden. Das Biomolekül kann beispielsweise Nukleinsäuren oder Derivate davon (DNA, RNA PNA, LNA, Oligonukleotide, Plasmide, Chromosomen), Peptide, Proteine (Enzym, Protein, Oligopeptide, zelluläre Rezeptorproteine und deren Komplexe, Peptidhormone, Antikörper und deren Fragmente), Kohlenhydrate und deren Derivate, insbesondere glykosylierte Proteine und Glycoside, Fette, Fettsäuren und/oder Lipide umfassen. Mit Hilfe der Clark-Elektrode kann der Sauerstoffgehalt im Bereich der Messoberfläche bestimmt werden, der Rückschlüsse auf die metabolische Aktivität der Zelle ermöglicht. Mittels eines IDES kann die Morphologie einer Zelle und mittels eines ISFET die Ansäuerung der Umgebung der Zelle gemessen werden. Mit Hilfe eines IDES und/oder ISFET können aber auch chemische Analysen durchgeführt werden. Es können also aktive und/oder passive Sensorelemente vorgesehen sein. Versorgungs- und Signalleitungen können vertikal durch die die Messoberfläche aufweidende Schicht verlaufen und/oder seitlich um diese herum angeordnet sein und mit Anschlüssen auf dem Substrat verbunden sein, so dass die Signale der Sensorelemente ausgelesen und/oder die aktiven Sensorelemente versorgt werden können.

Bei einer vorteilhaften Ausführungsform der Erfindung weist die Schicht an der Messoberfläche mindestens einen, für einen bestimmten Liganden bindungsspezifischen Rezeptor auf, wobei dem Rezeptor mindestens ein optischer Sensor zugeordnet ist, zum Detektieren einer in Abhängigkeit von der Bindung des Liganden an den Rezeptor erzeugten optischen Strahlung. Die Sensoranordnung kann dann zum Nachweisen und/oder Bestimmen der Konzentration des Liganden in einem zu untersuchenden Analyten verwendet werden.

Bei einer bevorzugten Ausgestaltung der Erfindung ist das mindestens eine Sensorelement in die mit dem Substrat lösbar verbindbare Schicht integriert, wobei vorzugsweise an der Schicht elektrische Kontakte und an dem Substrat damit zusammenwirkende Gegenkontakte zur Übertragung eines Messsignals von dem Sensorelement zu der Signalverarbeitungseinrichtung vorgesehen sind. Mit Hilfe des Sensors kann dann direkt an der Messoberfläche ein elektrisches oder optisches Signal detektiert und über die Kontakte und die Gegenkontakte an die in das Substrat integrierte Signalverarbeitungseinrichtung weitergeleitet werden. Es besteht aber auch die Möglichkeit, das Messsignal drahtlos von der Messoberfläche zu dem Substrat zu übertragen.

Bei einer anderen vorteilhaften Ausgestaltung der Erfindung ist das mindestens eine Sensorelement in das Substrat integriert. Dabei kann das Sensorelement als optisches Sensorelement ausgestaltet sein, das durch die die Messoberfläche aufweisende Schicht hindurch optische Strahlung empfangt.

Die die Messoberfläche aufweisende Schicht kann eine Folie aufweisen. Dabei ist es sogar möglich, dass die Folie aus einem Kunststoff besteht, der in einer orthogonal zur Erstreckungsebene der Folie verlaufenden Richtung eine größere elektrische Leitfähigkeit aufweist als in einer quer dazu verlaufenden Richtung. Dadurch können Kontakte zum elektrischen Verbinden der Folie mit dem Substrat besonders kostengünstig hergestellt werden. Femer können in die Oberfläche der Folie Vertiefungen eingeprägt sein, beispielsweise um die Folie gegen Verschmutzung unempfindlich zu machen (Lotusblüteneffekt).

Bei einer anderen vorteilhaften Ausgestaltung der Erfindung weist die Schicht ein Halbleitersubstrat auf Es ergibt sich dann ein Sandwhich-Aufbau, bei dem mehrere Halbleiterchips übereinander gestapelt sind. Dabei können die einzelnen Halbleiterchips mit unterschiedlichen Technologien hergestellt sein, beispielsweise als bipolare Silizium-Chips oder als CMOS-Chips.

Die Schicht kann mindestens ein Aktuatorelement aufweisen. Dadurch ist es beispielsweise möglich, eine an der Messoberfläche immobilisierte biologische Zelle zu stimulieren.

Vorteilhaft ist, wenn die Schicht benachbart zu dem mindestens einem Sensorelement und/oder dem mindestens einen Rezeptor ein verstellbares Diffusionshindernis für einen auf die Schicht aufbringbaren flüssigen Analyten aufweist, und wenn das Diffusionshindernis mit dem Aktuatorelement in Antriebsverbindung steht. Dadurch ist es insbesondere möglich, die Wahrscheinlichkeit, dass ein in dem Analyten befindlicher Ligand an einen an der Messoberfläche immobilisierten Rezeptor bindet, zu beeinflussen. Bei einer Messoberfläche, an der mehrere unterschiedliche Liganden immobilisiert sind, die für unterschiedliche, in unterschiedlicher Konzentration in dem Analyten enthaltene Rezeptoren bindungsspezifisch sind, kann dadurch die Diffusion im Bereich der einzelnen Rezeptoren so gesteuert werden, dass eine gleichmäßige Aussteuerung der einzelnen durch die Rezeptoren gebildeten Messstellen erreicht werden.

Bei einer bevorzugten Ausführungsform der Erfindung sind mehrere Sensorelemente matrixförmig vorzugsweise in mehreren Reihen und Spalten nebeneinander angeordnet. Dabei ist es sogar möglich mit Hilfe der Sensorelemente eine "elektronische Justierung" der Lage der die Messoberfläche aufweisenden Schicht relativ zu dem Substrat vorzunehmen, wenn der Oberflächenbereich, in dem sich die Sensorelemente erstrecken, größer ist als die Messoberfläche. Mit Hilfe der Signalverarbeitungseinrichtung können dann die einzelnen Messsignale aus den Sensorelementen ausgeglichen und miteinander und/oder einem Schwellwert verglichen werden, um das (die) Sensorelement(e) zu ermitteln, über dem (denen) die Messoberfläche positioniert ist Das Messsignal dieses Sensorelements kann dann ggf in der Signalverarbeitungseinrichtung weiter verarbeitet werden. Die die Messoberfläche aufweisende Schicht kann also mit relativ großen Lagetoleranzen auf dem Substrat positioniert werden, und dennoch kann das von der Messoberfläche ausgehende Signal zuverlässig mit den Sensorelementen detektiert und an die Signalverarbeitungseinrichtung weitergeleitet werden. Mögliche Anwendungen sind im Bereich der Mikrotiterplatte zu sehen.Hier ist es von Vorteil, wenn die elektrischen Kontakte, die aus der Schicht mit den Sensorelementen kommen, besonders groß sind (>500 µm).

Vorteilhaft ist, wenn die Durchgangslöcher jeweils mit einem Sensorelement korrespondierend angeordnet sind. Dabei ist es sogar möglich, dass die Sensorelemente ein optisches Sensor-Array bilden, von welchem jeweils ein Pixel einem Durchgangsloch zugeordnet ist. Die die Messoberfläche aufweisende Schicht kann dann noch besser auf dem Substrat fixiert werden.

Bei einer zweckmäßigen Ausgestaltung der Erfindung ist die die Messoberfläche aufweisende Schicht derart ausgebildet, dass eine weitere Schicht ablösbar auf ihr befestigt werden kann. Dabei kann die weitere Schicht ähnliche Eigenschaften aufweisen wie die erste. Die weitere Schicht kann eine Folie, ein Halbleiter- oder ein Glassubstrat sein. Auf den einzelnen Schichten können unterschiedliche Sensorelemente angeordnet sein, um unterschiedliche Eigenschaften nachzuweisen.

Die einzelnen Schichten und das Substrat können mit Hilfe unterschiedlicher Technologien hergestellt sein, z.B. kann eine erste Schicht als CMOS-Halbleiterchip mit Sensorik und Signalverarbeitungseinrichtung ausgestaltet sein. Eine zweite Schicht kann eine Folie sein, die eine Bedruckung aufweist. Ein solche lässt sich auf den CMOS-Halbleiterchip nicht aufbringen.

Zwischen der Schicht mit der Messoberfläche und dem Substrat kann eine weitere auswechselbare Schicht angeordnet sein, die Sensorelemente, Durchführungen zum Ansaugen und/oder elektrische Mittel für andere Arten zum beidseitigen reversiblen Anhaften tragen. Die zuerst genannte Schicht kann mit der weiteren Schicht so verbunden werden, dass zwischen den sensortragenden Schichten ein Kanal mit Sensoren gebildet ist, der von Flüssigkeiten und/oder Gasen durchströmt werden kann. Signal- und Versorgungsleitungen können durch die reversibel verbunden Schichten hindurch und/oder an diesen dem Substrat zugeführt sein. Die einzelnen Schichten können auch zu unterschiedlichen Zeitpunkten von dem Substrat entfernt werden.

Auf der die Sensorelemente aufweisenden Schicht können auch Sensorelemente angeordnet sein, die ihre Messwerte in Signale umwandeln, die von Sensoren auf und/oder in dem Substrat empfangen werden können. Dies kann dadurch geschehen, dass sich die Signale optisch, magnetisch, mechanisch und/oder elektrisch verändern, in Bewegung gesetzt werden, und/der Radiosignale abgeben.

Bei einer vorteilhaften Ausführungsform der Erfindung weist die Schicht in einem dem Sensorelement gegenüberliegenden Bereich mindestens einen Durchbruch auf Mit Hilfe des Sensorelements können dann durch den Durchbruch hindurch Messungen durchgeführt werden.

Vorteilhaft ist, wenn die Vorrichtung eine eine Innenhöhlung mit mindestens einer Einlassöffnung und wenigstens einer Auslassöffnung aufweisende Messkammer hat, die derart lösbar mit dem Substrat und/oder der Schicht verbindbar ist, dass diese an die Innenhöhlung der Messkammer angrenzt. Die Einlassöffnung und ggf die Auslassöffnung kann dann mit einem Fluidiksystem verbunden sein, über das ein zu analysierendes Fluid und/oder Wirkstoff zur Beeinflussung von in dem Fluid enthaltenen Biokomponenten auf einfache Weise der Messkammer zugeführt werden kann. Gegebenenfalls ist es sogar möglich, dass die Einlassöffnung und/oder die Auslassöffnung durch das Substrat hindurch geführt ist.

Auf dem Substrat können deutlich mehr Sensorelemente vorgesehen sein als Versorgungs- oder Signalleitungen auf der die Messoberfläche tragenden Schicht. Die für die tatsächliche Signalzuführung oder Beobachtung der Sensorelemente auf der Folie notwendigen Versorgungs- oder Signalleitungen können dann je nach Art und Lage der Schicht ausgewählt werden. Die Schicht kann so ausgebildet sein, dass sie Informationen über ihre Art und Lage auf dem Substrat abgibt, so dass eine adaptive Auswahl der richtigen Versorgungs- und Signalleitungen von einer Regel- oder Steuereinrichtung vorgenommen werden kann.

Erwähnt werden soll noch, dass sich auf der Folie zusätzlich zu dem Sensorelement auch eine Signalverarbeitungsvorrichtung befinden kann. Die Sensoranordnung kann dann z.B. in einem Touch-Screen verwendet werden, bei dem man die Oberfläche auswechseln will, weil sie z.B. verkratzt ist.

Vorteilhaft ist, wenn in und/oder auf der Schicht eine elektrische Energiequelle, insbesondere eine Batterie und/oder Brennstoffzelle angeordnet ist. Über die Energiequelle können dann das mindestens eine Sensorelement, der optische Sensor, ein optischer Sender zur Aussendung einer Anregungsstrahlung und/oder die Signalverarbeitungsvorrichtung mit Strom versorgt werden.

Nachfolgend sind Ausführungsbeispiele der Erfindung anhand der Zeichnung näher erläutert. Es zeigen:
- Fig. 1: einen Querschnitt durch eine Sensoranordnung, die ein Substrat aufweist, an das eine eine Messoberfläche aufweisende Schicht ansaugbar ist,
- Fig. 2: einen Querschnitt durch eine Sensoranordnung, die ein Substrat aufweist, auf dem eine eine Messoberfläche aufweisende Schicht magnetisch befestigbar ist,
- Fig. 3: eine Darstellung ähnlich Fig. 1, wobei jedoch die Schicht einen Durchbruch aufweist,
- Fig. 4: einen Querschnitt durch eine Sensoranordnung, auf der eine Schicht, die elektrische Kontaktelemente aufweist, auswechselbar fixiert ist und
- Fig. 5: einen Teilquerschnitt durch eine Sensoranordnung, bei der an der Messoberfläche verstellbare Diffusionshindernisse angeordnet sind.

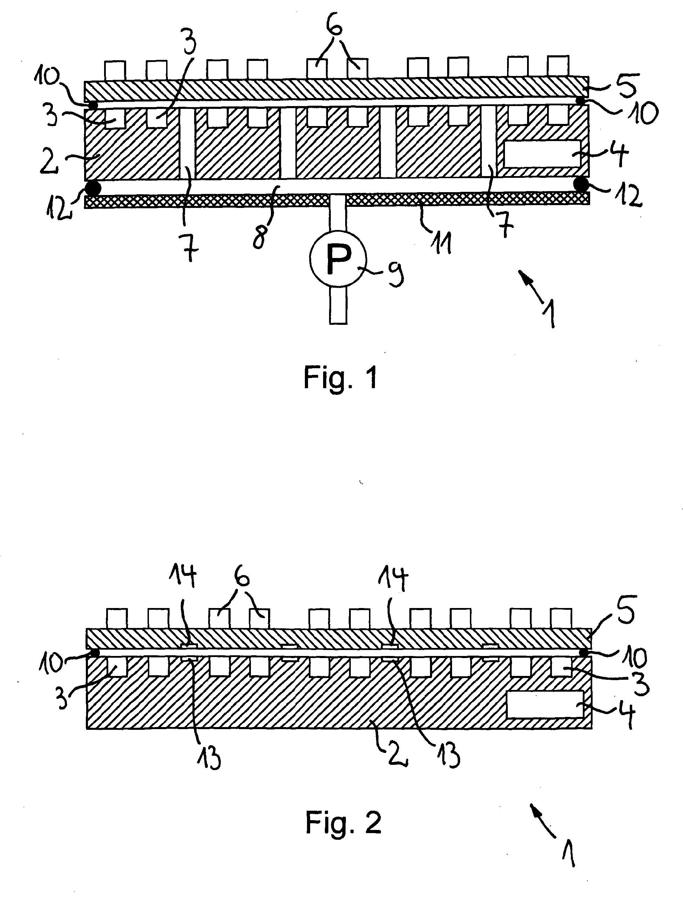

Eine in Fig. 1 im Ganzen mit 1 bezeichnete integrierte Sensoranordnung weist ein als Halbleiterchip ausgebildetes Substrat 2 auf, in das mehrere optische Sensorelemente 3 monolithisch integriert sind. Die Sensorelemente 3 sind matrixförmig in mehreren Reihen und Spalten nebeneinander angeordnet. In das Substrat 2 ist ferner eine Signalverarbeitungseinrichtung 4 integriert, die über in der Zeichnung nicht näher dargestellte Leiterbahnen mit den Sensorelementen 3 und Stromversorgungsanschlüssen verbunden ist.

Auf dem Substrat 2 ist etwa parallel zu dessen Erstreckungsebene eine Schicht angeordnet, die aus einer Folie 5 mit darauf angeordneten Rezeptoren 6 besteht., die jeweils für einen bestimmten, in einem mit der Messoberfläche in Kontakt bringbaren, zu untersuchenden Analyten enthaltenen Liganden bindungsspezifisch sind. An ihrer dem Substrat 2 abgewandten Vorderseite weist die Schicht eine Messoberfläche auf, an welche die Rezeptoren 6 angrenzen. In Abhängigkeit von der Bindung des Liganden an den Rezeptor 6 kann in an sich bekannter Weise eine optische Strahlung erzeugt werden, beispielsweise indem ein Rezeptor-Liganden-Komplex mit einem optischen Marker markiert wird, der bei Bestrahlung mit einer Anregungsstrahlung eine von der Wellenlänge der Anregungsstrahlung abweichende Wellenlänge aufweisende Lumineszenzstrahlung aussendet. Die Folie 5 ist für die Anregungsstrahlung durchlässig. In Fig. 1 ist erkennbar, dass die Rezeptoren 6 jeweils genau über einem ihnen jeweils zugeordneten Sensorelement 3 angeordnet sind.

Die aus der Folie 5 und den Rezeptoren 6 gebildete Schicht ist derart lösbar mit dem Substrat 2 verbunden, dass, wenn die Schicht mit dem Substrat 2 verbunden ist, ein von der Messoberfläche ausgehendes Signal von den Sensorelementen 3 empfangen werden kann und mit der Signalverarbeitungseinrichtung 4 nachweisbar ist.

Zum lösbaren Befestigen der Schicht auf dem Substrat 2 weist dieses mehrere seitlich voneinander beabstandete Durchgangslöcher 7 auf, die das Substrat 2 etwa normal zu seiner Erstreckungsebene durchsetzen. Jeweils ein erstes Ende jedes Durchgangslochs 7 ist der Rückseite der Schicht zugewandt und ein zweites Ende jedes Durchgangslochs 7 ist über einen Sammler 8 mit einem Absauganschluss einer den Durchgangslöchern 7 gemeinsam zugeordneten Pumpe 9 verbunden. Ein Auslassanschluss der Pumpe 9 ist mit der Atmosphäre verbunden. In Fig. 1 ist erkennbar, dass die Durchgangslöcher 7 jeweils zwischen den Sensorelementen angeordnet und seitlich von diesen beabstandet sind.

Die Folie 5 ist luftdicht ausgebildet und an ihrem Rand mittels eines Dichtrings 10 gegen das Substrat 2 abgedichtet. Es sind aber auch andere Ausführungsformen denkbar, bei denen die Folie 5 direkt an der Oberfläche des Substrats anliegen und gegen diese abdichten kann. In Fig. 1 ist zwischen der Folie 5 und Oberfläche des Substrats 2 ein schmaler Zwischenraum angeordnet, der sich parallel zur Ebene der Folie 5 erstreckt und durch den die Folie 5 von dem Substrat 2 beabstandet ist. Bei Beaufschlagung der Durchgangslöcher 7 mit einem Unterdruck kann sich der Abstand zwischen der Folie 5 und dem Substrat 2 bis auf null reduzieren.

Der Sammler 8 ist zwischen der der Folie 5 abgewandten Rückseite des Substrats 2 und einer das Substrat 2 überdeckenden Abdeckplatte 11 gebildet. Zwischen dem Substrat 2 und der Abdeckplatte 11 ist eine Dichtung 12 angeordnet, welche die Durchgangslöcher 7 umgrenzt. Eine mit dem Absauganschluss der Pumpe 9 verbundene Unterduckleitung durchsetzt die Abdeckplatte 11.

Zum Befestigen der Folie 5 auf dem Substrat 2 wird diese zunächst derart an dem Substrat 2 positioniert, dass die einzelnen, die Rezeptoren 6 aufweisenden Messstellen in der Aufsicht auf das Substrat 2 jeweils über einem ihnen zugeordneten Sensorelement 3 angeordnet sind und die Folie 5 gegen die Oberfläche des Substrats 2 abdichtet. Danach wird mit Hilfe der Pumpe 9 durch die Durchgangslöcher 7 hindurch an der Rückseite der Folie 5 ein Unterdruck angelegt, welcher die Folie 5 an das Substrat 2 andrückt.

Auch bei dem in Fig. 2 gezeigten Ausführungsbeispiel sind in ein Halbleiter-Substrat 2 mehrere optische Sensorelemente 3 und eine Signalverarbeitungseinrichtung 4 integriert. Letztere ist über in der Zeichnung nicht näher dargestellte Leiterbahnen mit den Sensorelementen 3 und Stromversorgungsanschlüssen verbunden.

Auf dem Substrat 2, ist etwa parallel zu dessen Erstreckungsebene eine Schicht lösbar angeordnet, die eine Folie 5 und eine Messoberfläche aufweist an der mehrere Messstellen vorgesehen sind, an denen für einen Liganden bindungsspezifische Rezeptoren 6 angeordnet sind.

Zum Fixieren der Folie 5 auf dem Substrat sind Mittel zum Erzeugen von magnetischen Kräften vorgesehen, die Permanentmagnete 13 und damit zusammenwirkende ferromagnetische Partikel 14 aufweisen. Die Permanentmagnete 13 sind an einem der Folie 5 zugewandten Oberflächenbereich des Substrats 2 angeordnet und die ferromagnetische Partikel 14 sind in die Rückseite der Folie 5 eingelassen. Wenn die Folie 5 auf dem Substrat positioniert ist, werden die ferromagnetischen Partikel 14 von dem magnetischen Fluss der Permanentmagnete 13 durchsetzt, wodurch die Folie 5 in einer Lage auf dem Substrat 2 fixiert wird, in der die einzelnen Rezeptoren 6 jeweils einem Sensorelement 3 gegenüberliegen. In Fig. 2 ist noch erkennbar, dass die Permanentmagnete 13 zwischen den Sensorelementen 3 angeordnet und seitlich von diesen beabstandet sind. Die ferromagnetischen Partikel 14 sind in der Aufsicht auf die Folie 5 zwischen den Rezeptoren 6 angeordnet.

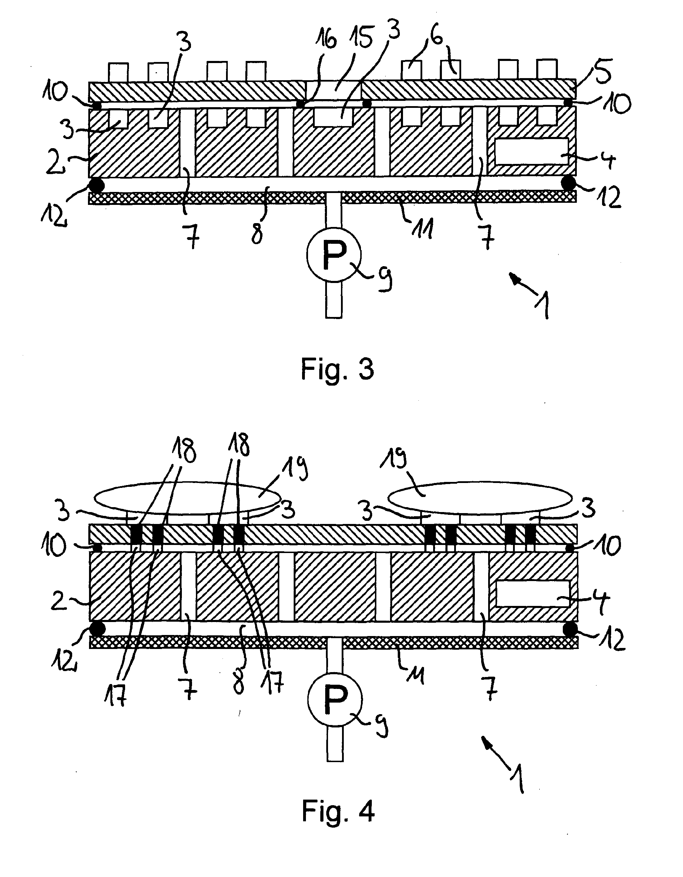

Der Aufbau eines in Fig. 3 gezeigten Ausführungsbeispiels der Sensoranordnung 1 entspricht weitgehend dem des Ausführungsbeispiels in Fig. 1, jedoch mit dem Unterschied, dass die Folie 5 einen Durchbruch 15 aufweist, hinter dem ein Sensorelement 3 angeordnet ist. Das Sensorelement 3 kann beispielsweise eine Clark-Elektrode sein. Der den Durchbruch 15 umgrenzende Randbereich der Folie 5 ist mittels eines um den Durchbruch 15 umlaufenden Dichtelements 16 gegen die Oberfläche des Substrats 2 abgedichtet.

Ein in Fig. 4 gezeigtes Ausführungsbeispiels der Sensoranordnung 1 weist ein Halbleiter-Substrat 2 auf, in das eine Signalverarbeitungseinrichtung 4 integriert ist, die über in der Zeichnung nicht näher dargestellte Leiterbahnen mit an einem der Folie 5 zugewandten Oberflächenbereich des Substrats 2 vorgesehenen elektrischen Gegenkontakten 17 verbunden ist. Wenn die Folie 5 auf dem Substrat 2 positioniert ist, kontaktieren die Gegenkontakte 17 jeweils einen die Folie 5 durchsetzenden Kontakt 18. An den Kontakten 18 sind Sensorelemente 3 angeschlossen. In Fig. 4 ist noch erkennbar, dass auf den Sensorelementen 3 lebende biologische Zellen 19 immobilisiert sind.

Zum lösbaren Befestigen der Folie 5 auf dem Substrat 2 weist dieses mehrere seitlich voneinander beabstandete Durchgangslöcher 7 auf, die das Substrat 2 quer zu seiner Erstreckungsebene durchsetzen. Jeweils ein erstes Ende jedes Durchgangslochs 7 ist der Rückseite der Folie 5 zugewandt und ein zweites Ende jedes Durchgangslochs 7 ist über einen Sammler 8 mit einem Absauganschluss einer den Durchgangslöchern 7 gemeinsam zugeordneten Pumpe 9 verbunden.

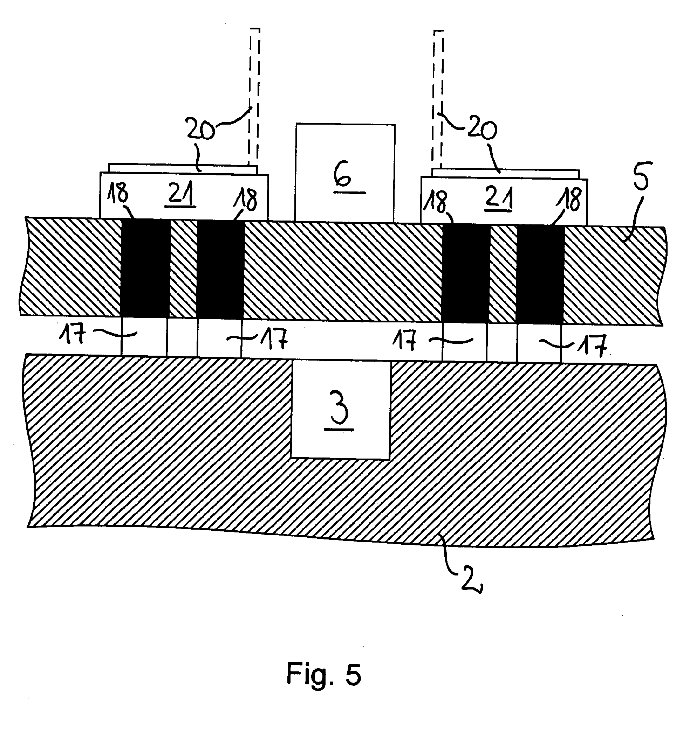

Bei dem in Fig. 5 gezeigten Ausführungsbeispiel weist die Schicht benachbart zu einem Rezeptor 6 verstellbare Diffusionshindernisse 20 für einen auf die Schicht aufbringbaren flüssigen Analyten auf Die Diffusionshindernisse 20 sind jeweils als flächige Elemente ausgebildet, die mit einem Aktuatorelement 21 in Antriebsverbindung stehen. Mittels der Aktuatorelemente 21 können die Diffusionshindernisse 20 aus einer Ruhelage, in der sie etwa parallel zur Erstreckungsebene der Folie 5 angeordnet sind, in eine in Fig. 5 strichliniert dargestellte Gebrauchslage verschwenkt werden, in der sie mit ihrer Ebene etwa rechtwinklig zu der Erstreckungsebene der Folie 5 verlaufen. In der Gebrauchslage schließen die Diffusionshindernisse 20 den Rezeptor 6 zwischen sich ein. Die Aktuatorelemente 21 sind über die Folie 5 durchsetzende elektrische Kontakte 18 mit auf dem Substrat 2 angeordneten Gegenkontakten 17 elektrisch verbunden. Die Gegenkontakte 17 sind zur Ansteuerung der Aktuatorelemente 21 mit der Signalverarbeitungseinrichtung 4 verbunden.

## Patentansprüche

1. Integrierte Sensoranordnung (1) mit einem Substrat (2), mindestens einem Sensorelement (3) und einer in das Substrat (2) integrierten, mit dem Sensorelement (3) verbundenen oder verbindbaren Signalverarbeitungseinrichtung (4), wobei eine eine Messoberfläche aufweisende Schicht derart lösbar mit dem Substrat (2) verbindbar ist, dass, wenn die Schicht mit dem Substrat (2) verbunden ist, ein von der Messoberfläche ausgehendes Signal mit der Signalverarbeitungseinrichtung (4) verarbeitbar ist, **dadurch gekennzeichnet, dass** das Substrat (2) mindestens ein an die Schicht angrenzendes Durchgangsloch (7) aufweist das zum Ansaugen der Schicht an das Substrat (2) mit einer Einrichtung zur Erzeugung eines Unterdrucks verbunden oder verbindbar ist.

2. Integrierte Sensoranordnung (1) nach Anspruch 1, **dadurch gekennzeichnet, dass** sich das mindestens eine Durchgangsloch (7) orthogonal zur Oberfläche des Substrates (2) erstreckt.

3. Integrierte Sensoranordnung (1) nach dem Oberbegriff von Anspruch 1, **dadurch gekennzeichnet, dass** sie Mittel zum Erzeugen von elektrostatischen und/oder magnetischen Kräften zum Fixieren der Schicht auf dem Substrat aufweist.

4. Integrierte Sensoranordnung (1) nach einem der Ansprüche 1 bis 3, **dadurch gekennzeichnet, dass** sie als Sensorelement mindestens einen für eine chemische und/oder physikalische Größe empfindlichen Sensor, insbesondere einen amperometrischen, potentiometrischen, magnetischen, optischen , Druck- und/oder Beschleunigungssensor aufweist.

5. Integrierte Sensoranordnung (1) nach einem der Ansprüche 1 bis 4, **dadurch gekennzeichnet, dass** die Schicht an der Messoberfläche mindestens einen, für einen bestimmten Liganden bindungsspezifischen Rezeptor (6) aufweist, und dass dem Rezeptor (6) mindestens ein optischer Sensor zugeordnet ist, zum Detektieren einer in Abhängigkeit von der Bindung des Liganden an den Rezeptor (6) erzeugten optischen Strahlung.

6. Integrierte Sensoranordnung (1) nach einem der Ansprüche 1 bis 5, **dadurch gekennzeichnet, dass** das mindestens eine Sensorelement (3) in die mit dem Substrat (2) lösbar verbindbare Schicht integriert ist, und dass vorzugsweise an der Schicht elektrische Kontakte (18) und an dem Substrat (2) damit zusammenwirkende Gegenkontakte (19) zur Übertragung eines Messsignals von dem Sensorelement (3) zu der Signalverarbeitungseinrichtung (4) vorgesehen sind.

7. Integrierte Sensoranordnung (1) nach einem der Ansprüche 1 bis 6, **dadurch gekennzeichnet, dass** das mindestens eine Sensorelement (3) in das Substrat (2) integriert ist.

8. Integrierte Sensoranordnung (1) nach einem der Ansprüche 1 bis 7, **dadurch gekennzeichnet, dass** die Schicht eine Folie (5) aufweist die vorzugsweise für eine Wellenlänge durchlässig ist, für die der optische Sensor empfindlich ist.

9. Integrierte Sensoranordnung (1) nach einem der Ansprüche 1 bis 8, **dadurch gekennzeichnet, dass** die Folie (5) aus einem Kunststoff besteht, der in einer orthogonal zur Erstreckungsebene der Folie (5) verlaufenden Richtung eine größere elektrische Leitfähigkeit aufweist als in einer quer dazu verlaufenden Richtung.

10. Integrierte Sensoranordnung (1) nach einem der Ansprüche 1 bis 9, **dadurch gekennzeichnet, dass** die Schicht ein Halbleitersubstrat aufweist

11. Integrierte Sensoranordnung (1) nach einem der Ansprüche 1 bis 10, **dadurch gekennzeichnet**, die Schicht mindestens ein Aktuatorelement (21) aufweist

12. integrierte Sensoranordnung (1) nach einem der Ansprüche 1 bis 11, **dadurch gekennzeichnet, dass** die Schicht benachbart zu dem mindestens einem Sensorelement und/oder dem mindestens einen Rezeptor ein verstellbares Diffusionshindernis für einen auf die Schicht aufbringbaren flüssigen Analyten aufweist, und dass das Diffusionshindernis (20) mit dem Aktuatorelement (21) in Antriebsverbindung steht.

13. Integrierte Sensoranordnung (1) nach einem der Ansprüche 1 bis 12, **dadurch gekennzeichnet, dass** mehrere Sensorelemente (3) matrixförmig vorzugsweise in mehreren Reihen und Spalten nebeneinander angeordnet sind.

14. Integrierte Sensoranordnung (1) nach einem der Ansprüche 1 bis 13, **dadurch gekennzeichnet, dass** die Durchgangslöcher (7) jeweils mit einem Sensorelement (3) korrespondierend angeordnet sind.

15. Integrierte Sensoranordnung (1) nach einem der Ansprüche 1 bis 14, **dadurch gekennzeichnet, dass** die Schicht derart ausgebildet ist, dass eine weitere Schicht ablösbar auf ihr befestigt werden kann.

16. Integrierte Sensoranordnung (1) nach einem der Ansprüche 1 bis 15, **dadurch gekennzeichnet, dass** die Schicht in einem einem Sensorelement (3) gegenüberliegenden Bereich mindestens einen Durchbruch (15) aufweist.

17. Integrierte Sensoranordnung (1) nach einem der Ansprüche 1 bis 16, **dadurch gekennzeichnet, dass** sie eine eine Innenhöhlung mit mindestens einer Einlassöffnung und wenigstens einer Auslassöffnung aufweisende Messkammer hat, die derart lösbar mit dem Substrat (2) und/oder der Schicht verbindbar ist, dass diese an die Innenhöhlung der Messkammer angrenzt.

18. Integrierte Sensoranordnung (1) nach einem der Ansprüche 1 bis 17, **dadurch gekennzeichnet, dass** in und/oder auf der Schicht eine elektrische Energiequelle, insbesondere eine Batterie und/oder Brennstoffzelle angeordnet ist.
